# EUROPEAN PATENT APPLICATION

(11) **EP 2 289 473 A2**
(43) Date of publication of application: **02.03.2011**
(21) Application number: 10251391.8
(22) Date of filing: 04.08.2010
(51) Int. Cl.: A61F 13/15

(54) **Aborbent article including absorbent core having a plurality of first regions and a second region having differnt basis weight surrounding each of the first regions**

(30) Priority: 05.08.2009 US 535752
(71) Applicant: Johnson & Johnson Industria e Comercio Ltda., 12237-350 Sao Paulo (BR)
(72) Inventor: Ribeiro de Carvalho, Antonio Carlos, Taubate Sao Paulo 12020-260 (BR); Caldas Salles, Maria Marcia R., San Jose dos Campos Sao Paulo 12243-680 (BR); Teixeira Fajolli, Marcia Helena, Mog das Cruzes Sao Paulo 08735-420 (BR)
(74) Representative: Kirsch, Susan Edith

(57) **Abstract**

An absorbent article including an absorbent core having a plurality of first regions and a second region, each one of said first regions being arranged in spaced relationship from each of the other first regions and each of the first regions being entirely surrounded by the second region.

## Description

### FIELD OF THE INVENTION

The present invention generally relates to sanitary absorbent articles and in particular to feminine sanitary absorbent napkins having enhanced breathability, temperature and humidity control characteristics.

### BACKGROUND OF THE INVENTION

Externally worn, sanitary absorbent napkins are one of many kinds of feminine protection devices currently available. Sanitary napkins conventionally have a laminate construction including a body-facing liquid permeable layer, an absorbent core layer or layers, and a liquid impermeable garment facing layer. A problem with conventional napkins, due to the laminate construction thereof, is that such articles are not particularly breathable within the absorbent layers of the article. This lack of "internal breathability" within the article construct can cause comfort problems for the user during use of the article. In particular, the lack of internal breathability in conventional articles may cause the users body temperature to rise in a localized area thereby creating discomfort during use. Further, once the article becomes wet, the lack of internal breathability may prevent the article from drying thereby imparting a wet sensation to the user during use.

The inventors of the present invention have discovered a sanitary napkin construction that overcomes the shortcomings of conventional sanitary napkins described above and more particularly have disclosed herein a napkin construction that provides enhanced breathability, temperature and humidity control characteristics.

### SUMMARY OF THE INVENTION

In view of the foregoing, the present invention provides an absorbent article including a liquid pervious cover layer, a liquid impervious barrier layer, an absorbent core arranged between the cover layer and barrier layer, the absorbent core having a plurality of first regions and a second region, each one of said first regions being arranged in spaced relationship from each of the other first regions and each of the first regions being entirely surrounded by the second region, the second region having a basis weight and each one of the plurality of first regions having a basis weight, wherein the basis weight of each of the first regions is less than the basis weight of the second region, wherein the entire absorbent core, including the plurality of first regions and the second region, has a uniform thickness.

### BRIEF DESCRIPTION OF THE DRAWINGS

Examples of embodiments of the present invention will now be described with reference to the drawings, in which:
Fig. 1 is a perspective view of a sanitary napkin in accordance with an embodiment of the present invention;
Fig. 2 is an exploded view of the sanitary napkin shown in Fig. 1, according to a first embodiment of the invention, showing the constituent layers thereof;
Fig. 3 is an exploded view of the sanitary napkin shown in Fig. 1, according to a second embodiment of the invention, showing the constituent layers thereof;
Fig. 4 is a top plan view of the core layer of the sanitary napkin shown in Fig. 1 showing the first and second concentric regions thereof;
Fig. 5 is a sectional view taken along line 5-5 in Fig. 4;
Fig. 6 is a sectional view taken along line 6-6 in Fig. 4;
Fig. 7 is a sectional view taken along line 7-7 in Fig. 4;
Fig. 8 is a schematic view showing an apparatus for making the core layer shown in Figs. 3-7;
Fig. 9 is a detailed perspective view of a portion of the apparatus shown in Fig. 8;
Fig. 10 is a sectional view of the apparatus shown in Fig. 8 taken along line 10-10 thereof;
Fig. 11 is a detailed elevation view of a portion of the apparatus shown in Fig. 8;
Fig. 12 is a detailed perspective view of a portion of the apparatus shown in Fig. 8;
Fig. 13 is a schematic view of an apparatus for measuring relative humidity of an absorbent article;
Fig. 14 is a perspective view of an absorbent article according to the present invention with the temperature and relatively humidity microsensors of the apparatus depicted in Fig. 13 inserted under the cover layer and into the core layer thereof;
Fig. 14a is a detailed sectional view of the absorbent article shown in Fig. 14 depicting the insertion of the temperature and relatively humidity microsensors into the core layer of the absorbent article;
Fig. 15 is a partially exploded view depicting additional features of the apparatus shown in Fig. 13;
Fig. 16 is a perspective view showing the absorbent article positioned for testing in the apparatus shown in Fig. 15;
Fig. 17 is a graph plotting relatively humidity versus time for an absorbent article in accordance with the present invention tested according to the "Procedure for Measuring Relative Humidity" test method set forth herein;
Fig. 18 is a graph depicting the differential plot of the graph shown in Fig. 17;
Fig. 19 is a graph showing the manner in which X₁ and Y₁ are determined according to Procedure for Measuring Relative Humidity test method;
Fig. 20 is a graph showing how a first tangent line is determined for the graph shown in Fig. 17 based on X₁ and Y₁;
Figs. 21 and 22 are graphs depicting the manner in which a second tangent line is determined for the graph shown in Fig. 17;
Fig. 23 is graph depicting the manner in which A_{RH} is calculated based upon the first and second tangent lines for the absorbent article according to the present invention; and

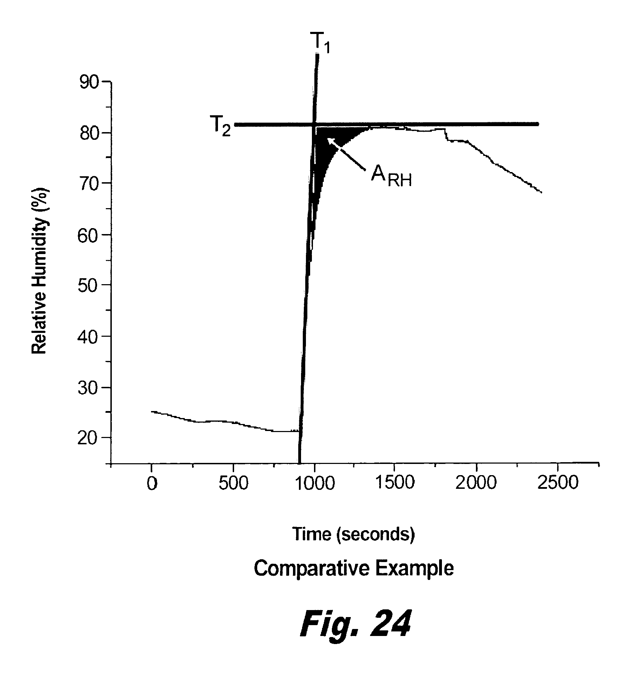

Fig. 24 is graph depicting A_{RH} for a comparative product example.

### DETAILED DESCRIPTION OF THE INVENTION

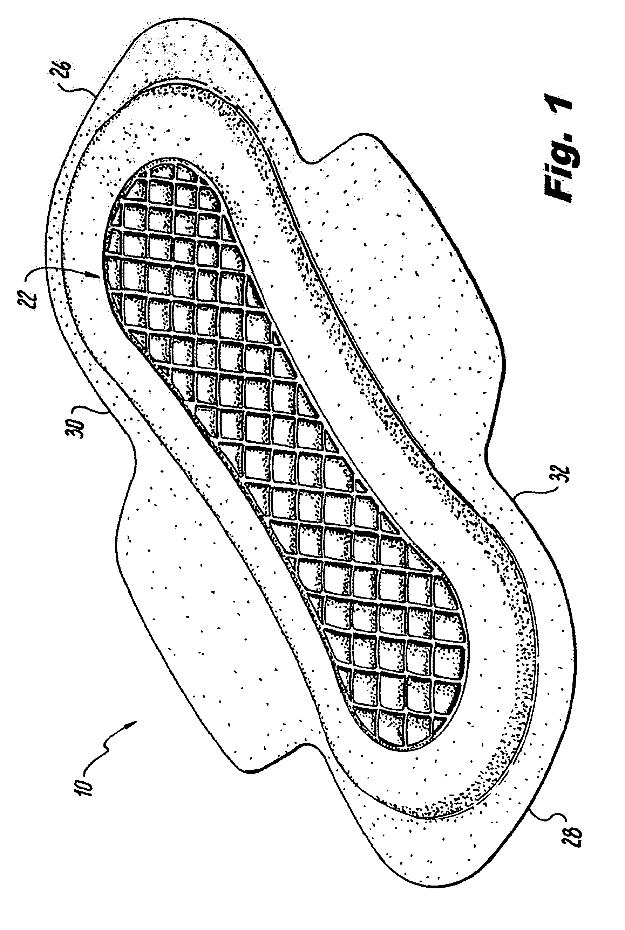

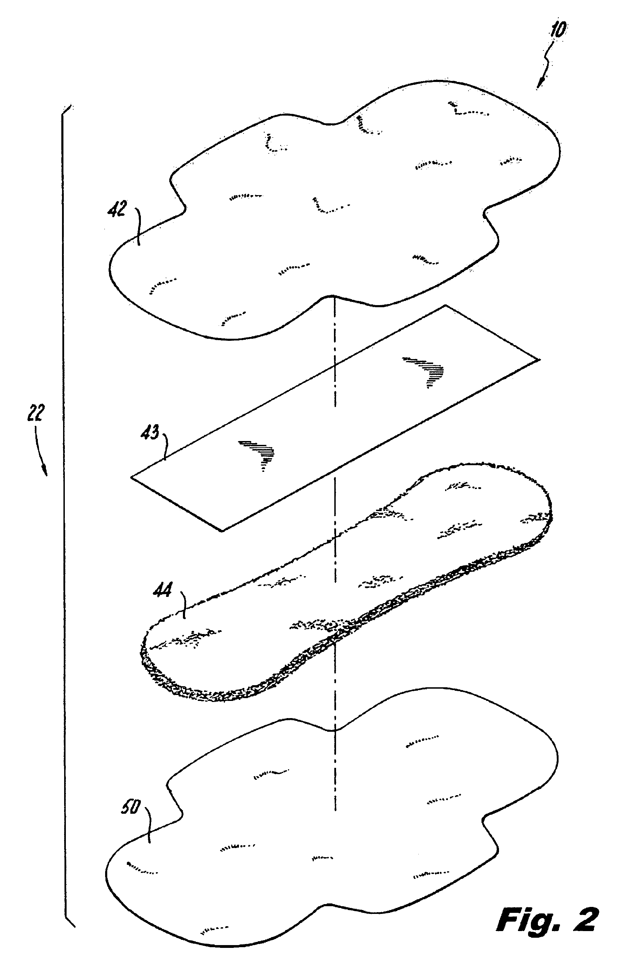

Referring to FIGS. 1 and 2, there is shown an embodiment of the present invention, a feminine sanitary napkin 10.

The sanitary napkin 10 has a main body 22 with a first transverse side 26 defining a front portion thereof and a second transverse side 28 defining a rear portion thereof. The main body also has two longitudinal sides, namely a longitudinal side 30 and a longitudinal side 32.

As depicted in FIG. 2, the main body 22, according to a first embodiment of the invention, is of a laminate construction and includes a fluid-permeable cover layer 42, a transfer layer 43, an absorbent core 44 and a fluid-impervious barrier layer 50.

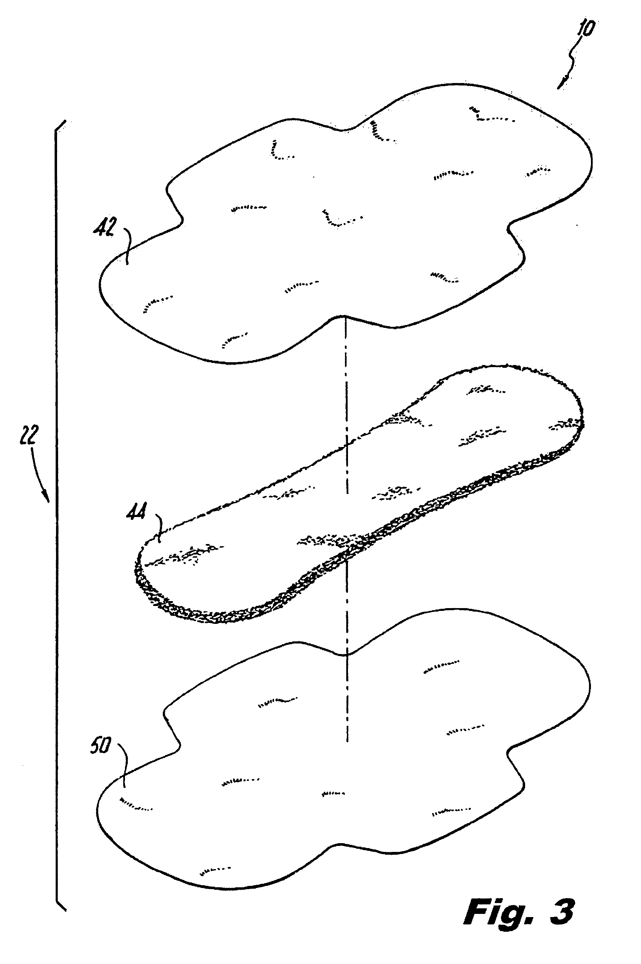

As depicted in FIG. 3, the main body 22, according to a second embodiment of the invention, is of a laminate construction and includes a fluid-permeable cover layer 42, an absorbent core 44, and a fluid-impervious barrier layer 50.

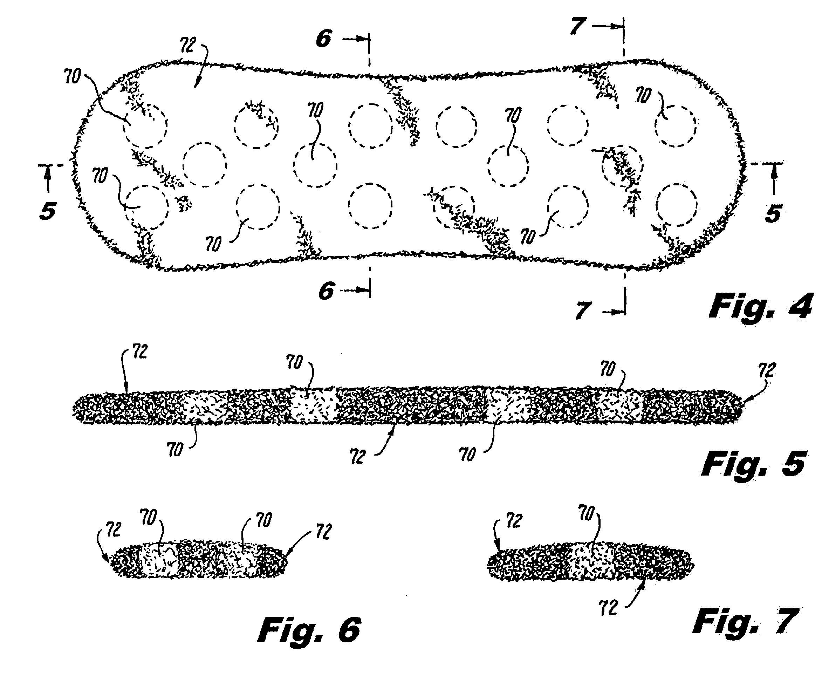

Referring to Figs. 4-7, the absorbent core 44 includes a plurality of first regions 70 and a second region 72. As shown in Fig. 4, each of the plurality of first regions 70 are arranged in spaced relationship to one another and are entirely surrounded by the second region 72.

Each of the plurality of first regions 70 has a basis weight in the range of between 7.5 gsm (g/m²) and 555 gsm and the second region 72 has a basis weight in the range of between 150 gsm and 650 gsm. The basis weight of each of the plurality of first regions 70 is selected such that is less than the basis weight of the second region 72. In particular the basis weight of each of the plurality of first regions 70 is selected such that each region 70 has a basis weight of about 5% to about 85% the basis weight of the second region 72. In one preferred embodiment of the invention the basis weight of each of the plurality of first regions 70 is selected such that each region 70 has a basis weight of about 50% the basis weight of the second region 72.

Each of the plurality of first regions 70 has a density that is less than the density of the second region. Specifically each of the first regions 70 preferably has a density in the range from about 0.017 g/cm³ to 0.200 g/cm³ and the second region 72 has a density in the range of about 0.035 g/cm³ to 0.400 g/cm³. In one preferred embodiment of the invention each of the first regions 70 has a density that is less than 80 % the density of the second region 72, and more preferably less than 50%.

While in one preferred embodiment of the invention each of the first regions 70 have the same density, it is possible that individual first regions 70 have a different densities from one another provided that each of the first regions 70 has a density less than the density of the second region 72.

Preferably the plurality of first regions 70 (i.e. the summation of the area over which the first regions extend) extend over about 5% to about 30% the surface area of the core 44 and the second region 72 extends over about 70% to 95% of the core. The absorbent core 44, including those areas defined by the plurality of first regions 70 and the second region 72, preferably has a uniform thickness between about 0.5 mm and about 12 mm. In a preferred embodiment of the invention, the absorbent core 44 comprises between about 75% to 100% cellulose fibers by weight and 0% to 25% superabsorbent polymer by weight. In a particularly preferred embodiment, the plurality of first regions 70 and the second region have 72 the same identical material composition. Also, preferably, the plurality of first regions 70 and second region 72 are composed of a single layer of material, that is the plurality of first regions 70 and second region 72 are not formed by layering two distinct layers one on top of another.

### Main Body--Cover Layer

The cover layer 42 may be a relatively low density, bulky, high-loft non-woven web material. The cover layer 42 may be composed of only one type of fiber, such as polyester or polypropylene or it may include a mixture of more than one fiber. The cover may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. Preferably, the cover layer 42 has a basis weight in the range of about 10 gsm to about 75 gsm.

Bi-component fibers may be made up of a polyester layer and a polyethylene sheath. The use of appropriate bi-component materials results in a fusible non-woven fabric. Examples of such fusible fabrics are described in U.S. Pat. No. 4,555,430 issued Nov. 26, 1985 to Chicopee. Using a fusible fabric increases the ease with which the cover layer may be mounted to the absorbent layer and/or to the barrier layer.

The cover layer 42 preferably has a relatively high degree of wettability, although the individual fibers comprising the cover may not be particularly hydrophilic. The cover material should also contain a great number of relatively large pores. This is because the cover layer 42 is intended to take-up body fluid rapidly and transports it away from the body and the point of deposition. Therefore, the cover layer contributes little to the time taken for the napkin to absorb a given quantity of liquid (penetration time).

Advantageously, the fibers that make up the cover layer 42 should not lose their physical properties when they are wetted, in other words they should not collapse or lose their resiliency when subjected to water or body fluid. The cover layer 42 may be treated to allow fluid to pass through it readily. The cover layer 42 also functions to transfer the fluid quickly to the underlying layers of the absorbent article. Thus, the cover layer 42 is advantageously wettable, hydrophilic and porous. When composed of synthetic hydrophobic fibers such as polyester or bi-component fibers, the cover layer 42 may be treated with a surfactant to impart the desired degree of wettability.

In one preferred embodiment of the present invention the cover is made from a 16 gsm thermal bonded polypropylene fiber nonwoven of the type commercially available from Polystar Company, Salvador, BA , Brazil under product code 142250.

Alternatively, the cover layer 42 can also be made of polymer film having large pores. Because of such high porosity, the film accomplishes the function of quickly transferring body fluid to the underlying layers of the absorbent article. A suitable cover material of this type is commercially found on the STAYFREE Dry Max Ultrathin product distributed by McNeil-PPC, Inc.

The cover layer 42 may be embossed to the remainder of the absorbent core 44 in order to aid in promoting hydrophilicity by fusing the cover to the next layer. Such fusion may be effected locally, at a plurality of sites or over the entire contact surface of cover layer 42 and absorbent core 44. Alternatively, the cover layer 42 may be attached to the absorbent core 44 by other means such as by adhesion.

### Main Body -- Transfer Layer

Adjacent to the cover layer 42 on its inner side and bonded to the cover layer 42 is the transfer layer 43. The transfer layer 43 provides the means of receiving body fluid from the cover layer 42 and holding it until the underlying absorbent core 44 has an opportunity to absorb the fluid, and therefore acts as a fluid transfer or acquisition layer. The transfer layer 43 is, preferably, more dense than and has a larger proportion of smaller pores than the cover layer 42. These attributes allow the transfer layer 43 to contain body fluid and hold it away from the outer side of the cover layer 42, thereby preventing the fluid from rewetting the cover layer 42 and its surface. However, the transfer layer is, preferably, not so dense as to prevent the passage of the fluid through the layer 43 into the underlying absorbent core 44.

The transfer layer 43 be composed of fibrous materials, such as wood pulp, polyester, rayon, flexible foam, or the like, or combinations thereof. The transfer layer 43 may also comprise thermoplastic fibers for the purpose of stabilizing the layer and maintaining its structural integrity. The transfer layer 43 may be treated with surfactant on one or both sides in order to increase its wettability, although generally the transfer layer 43 is relatively hydrophilic and may not require treatment. The transfer layer 43 is preferably bonded or adhered on both sides to the adjacent layers, i.e. the cover layer 42 and the underlying absorbent core 44.

Examples of suitable materials for the transfer layer are through air bonded pulp sold by Buckeye of Memphis, Tenn., under the designation VIZORB 3008, which has a basis weight of 110gsm, VIZORB 3042, which has a basis weight of 100gsm, VIZORB 3010, which has a basis weight of 90gsm and others.

### Main Body -- Absorbent Core

In one preferred embodiment of the invention, the absorbent core 44 is a blend or mixture of cellulosic fibers and superabsorbent disposed therein. Cellulosic fibers that can be used in the absorbent core 44 are well known in the art and include wood pulp, cotton, flax and peat moss. Wood pulp is preferred. Pulps can be obtained from mechanical or chemi-mechanical, sulfite, kraft, pulping reject materials, organic solvent pulps, etc. Both softwood and hardwood species are useful. Softwood pulps are preferred. It is not necessary to treat cellulosic fibers with chemical debonding agents, cross-linking agents and the like for use in the present material. Some portion of the pulp may be chemically treated as discussed in U.S. Pat. No. 5,916,670 to improved flexibility of the product. The flexibility of the material may also be improved by mechanically working the material or tenderizing the material.

The absorbent core 44 can contain any superabsorbent polymer (SAP), which are well known in the art. For the purposes of the present invention, the term "superabsorbent polymer" (or "SAP") refers to materials, which are capable of absorbing and retaining at least about 10 times their weight in body fluids under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. Of Osaka, Japan, under the designation of SA70N and products offered by Stockhausen Inc.

In one preferred embodiment of the invention the absorbent core 44 includes between 50% and 100% cellulose pulp by weight and 0% and 50% superabsorbent polymer by weight.

In one specific example of the invention, the absorbent core 44 is constructed from about 93 % fluff pulp by weight, suitable pulp commercially available as Golden Isles Fluff Pulp 420#HD 7% Moisture, from GP Cellulose, Brunswick, Georgia, USA, mixed with about 7% superabsorbent polymer by weight, suitable SAP commercially available as Aqua Keep SA70N from Sumitomo Seika Chemicals Co., Ltd., Osaka, Japan.

### Method of Making the Absorbent Core

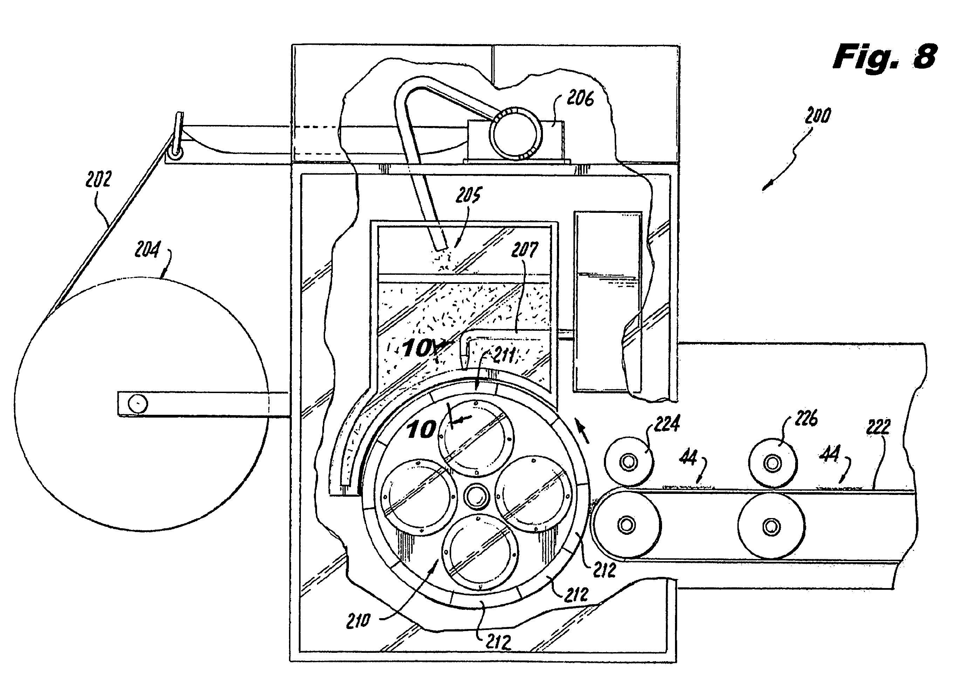

A description of the method of making the absorbent core according to the present invention will now be provided with reference to Figs. 8-12 which depict an apparatus 200 for making the absorbent core structure according to the present invention. The pulp used to form the absorbent core 44 is a bleached softwood pulp, produced by a Kraft process. The pulp is provided by the manufacturer as a pulp board 202 in rolled form, the roll identified by the reference numeral 204 in Fig. 8. The pulp board 202 is conveyed from the roll 204 to a device 206 for grinding the pulp board 202 into fibrous pulp 205. The fibrous pulp 205 is released from the grinding device 206 into a chamber 208 for holding the fibrous pulp 205. The apparatus 200 may further optionally include a device 207 for introducing superabsorbent polymer into the chamber 208 to thereby form a fibrous pulp and superabsorbent mixture. Any conventional device suitable for this purpose, and known to those of skill in the art, may be used for introducing the superabsorbent into the chamber 208.

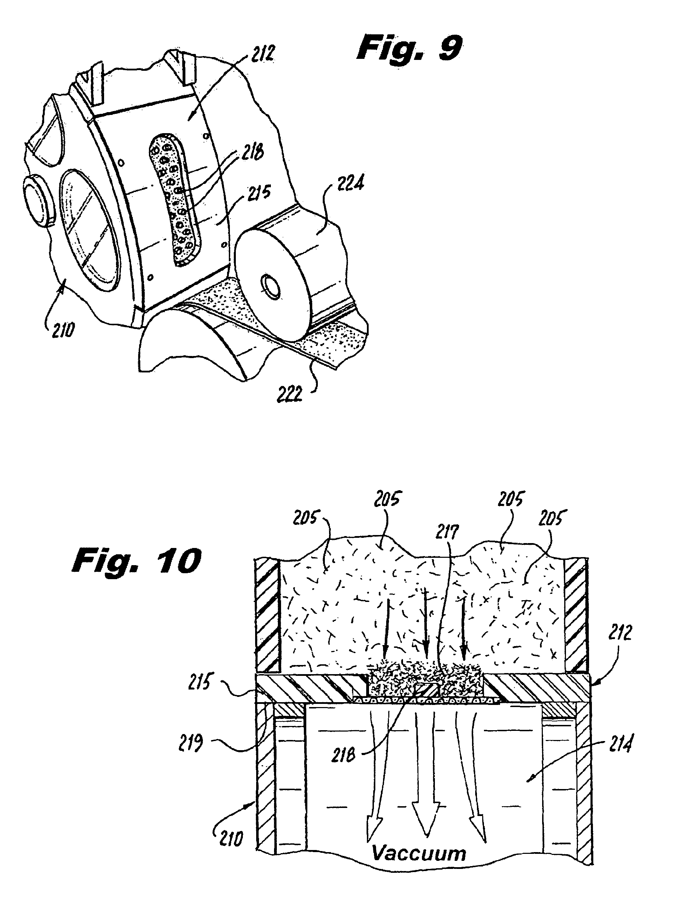

The chamber 208 has a partially open bottom portion 211 that communicates with a rotating forming drum 210. The rotating forming drum 210 has a plurality of molds 212 mounted thereto. As the forming drum 210 rotates, each of the molds 212 are sequentially arranged in communication with the open portion 211 of the chamber 208 to thereby receive fibrous pulp 205 from the chamber 208. In Figure 8, the forming drum 210 rotates in a counterclockwise manner during operation of the apparatus 200. As shown in Fig. 10, the forming drum 210 includes a portion 214 that is under vacuum. As shown in Figs. 9 and 10, the mold 212 includes a porous screen 217 structure in the shape of the second region 72 of the core 44. As the mold 212 passes over portion 214 of the forming drum 210 the vacuum functions to draw the fibrous pulp 205 from the chamber 208 into the mold 212 by drawing air through the porous screen 217 of the mold 212.

As shown in detail in Fig. 10, the mold 212 includes a nonporous mounting plate portion 215 that surrounds the porous screen 217 portion of the mold 212. As best seen in Fig. 10, the mounting plate portion 215 of the mold 212 is mounted to the periphery 219 of the forming drum 210, thereby enabling each of the molds 212 to rotate with the rotating forming drum 210. The porous screen 217 portion of the mold 212 is arranged in the shape of the second region 72 of the core 44. The mold 212 further includes a plurality of nonporous projections 218, each projection 218 being in the shape of one of the plurality of first regions 70. Each of nonporous projections 218 has a height that is less than the height of the mounting plate portion 215. The structure of the mold 212 described above, during use, causes a greater amount of fibrous pulp 205 to be drawn into the porous screen 217 portion of the mold 212 relative to the amount of fibrous pulp 205 drawn into each nonporous projections 218.

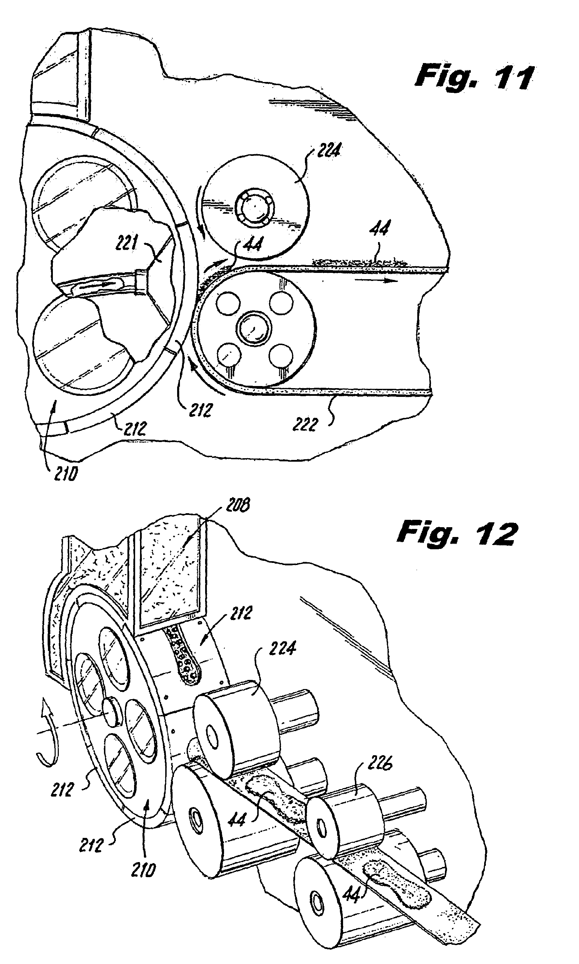

After the mold 212 is rotated under the partially open bottom portion 211 of the chamber 208, the mold 212 is further rotated by the rotating forming drum 210. As shown in Fig. 11, the rotating forming drum 210 includes a portion 221 that expels air outwardly from within the drum 210. The portion 221 of the drum 210 functions to expel the core 44 structure formed within the mold 212 onto a belt 222. The belt 222 functions to convey the core 44 to a first calendar roll 224. The calendar roll 224 functions to reduce the thickness of the core 44 so that the first 70 and second 72 regions of the core 44 have a uniform thickness but have different basis weights. As shown in Fig. 12, the core 44 may optionally be further conveyed by the belt 222 to a second calendar roll 226 that functions to further reduce the thickness of the core 44.

### Main Body-Barrier Layer

Underlying the absorbent core 44 is a barrier layer 50 comprising liquid-impervious film material so as to prevent liquid that is entrapped in the absorbent core 44 from egressing the sanitary napkin and staining the wearer's undergarment. The barrier layer 50 is preferably made of polymeric film, although it may be made of liquid impervious, air-permeable material such as repellent-treated non-woven or micropore films or foams.

The barrier layer may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet. The cover layer 42 and the barrier layer 50 are joined along their marginal portions so as to form an enclosure or flange seal that maintains the absorbent core 44 captive. The joint may be made by means of adhesives, heat-bonding, ultrasonic bonding, radio frequency sealing, mechanical crimping, and the like and combinations thereof.

Positioning adhesive may be applied to a garment facing side of the barrier layer for securing the napkin 10 to the garment during use. The positioning adhesive may be covered with removable release paper so that the positioning adhesive is covered by the removable release paper prior to use.

Absorbent articles of this invention may or may not include wings, flaps or tabs for securing the absorbent article to an undergarment. Wings, also called, among other things, flaps or tabs, and their use in sanitary protection articles is described in U.S. Patent. No. 4,687,478 to Van Tilburg; U.S. Patent No. 4,589,876 also to Van Tilburg, U.S. Patent No. 4,900,320 to McCoy, and U.S. Patent No. 4,608,047 to Mattingly. The disclosures of these patents are incorporated herein by reference in their entirety. As disclosed in the above documents, wings are generally speaking flexible and configured to be folded over the edges of the underwear so that the wings are disposed between the edges of the underwear.

The absorbent article of the present invention may be applied to the crotch by placing the garment-facing surface against the inside surface of the crotch of the garment. Various methods of attaching absorbent articles may be used. For example, chemical means, e.g., adhesive, and mechanical attachment means, e.g., clips, laces, ties, and interlocking devices, e.g., snaps, buttons, VELCRO (Velcro USA, Inc., Manchester, NH), zipper, and the like are examples of the various options available to the artisan.

Adhesive may include pressure sensitive adhesive that is applied as strips, swirls, or waves, and the like. As used herein, the term pressure-sensitive adhesive refers to any releasable adhesive or releasable tenacious means. Suitable adhesive compositions include, for example, water-based pressure-sensitive adhesives such as acrylate adhesives. Alternatively, the adhesive composition may include adhesives based on the following: emulsion or solvent-borne adhesives of natural or synthetic polyisoprene, styrene-butadiene, or polyacrylate, vinyl acetate copolymer or combinations thereof; hot melt adhesives based on suitable block copoylmers - suitable block copolymers for use in the invention include linear or radial co-polymer structures having the formula (A-B)x wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalpha-methylstyrene, Polyvinyltoluene, and combinations thereof. Suitable Block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof. Commercial examples of these types of block copolymers include KratonTM elastomers from Shell Chemical Company, VectorTM elastomers from Dexco, SolpreneTM from Enichem Elastomers and StereonTM from Firestone Tire & Rubber Co.; hot melt adhesive based on olefin polymers and copolymers where in the olefin polymer is a terpolymer of ethylene and a co-monomers, such as vinyl acetate, acrylic acid, methacrylic acid, ethyl acrylate, methyl acrylate, n-butyl acrylate vinyl silane or maleic anhydride. Commercial examples of these types of polymers include Ateva( polymers from AT plastics), Nucrel( polymers from DuPont), Escor (from Exxon Chemical).

Where adhesive is used, a release strip may be applied to protect the adhesive on the absorbent article prior to attaching the absorbent article to the crotch. The release strip can be formed from any suitable sheet-like material adheres with sufficient tenacity to the adhesive to remain in place prior to use but which can be readily removed when the absorbent article is to be used. Optionally, a coating may be applied to release strip to improve the ease of removabilty of the release strip from the adhesive. Any coating capable of achieving this result may be used, e.g., silicone.

Any or all of the cover, absorbent layer, transfer layer, backsheet layer, and adhesive layers may be colored. Such coloring includes, but is not limited to, white, black, red, yellow, blue, orange, green, violet, and mixtures thereof. Color may be imparted according to the present invention through dying, pigmentation, and printing. Colorants used according the present invention include dyes and inorganic and organic pigments. The dyes include, but are not limited to, anthraquinone dyes (Solvent Red 111, Disperse Violet 1, Solvent Blue 56, and Solvent Green 3), Xanthene dyes (Solvent Green 4, Acid Red 52, Basic Red 1, and Solvent Orange 63), azine dyes (Jet black), and the like. Inorganic pigments include, but are not limited to, titanium dioxide (white), carbon black (black), iron oxides (red, yellow, and brown), chromium oxide (green), ferric ammonium ferrocyanide (blue), and the like.

Organic pigments include, but are not limited to diarylide yellow AAOA (Pigment Yellow 12), diarylide yellow AAOT (Pigment Yellow 14), phthalocyanine blue (Pigment Blue 15), lithol red (Pigment Red 49:1), Red Lake C (Pigment Red), and the like.

The absorbent article may include other known materials, layers, and additives, such as, foam, net-like materials, perfumes, medicaments or pharmaceutical agents, moisturizers, odor control agents, and the like. The absorbent article can optionally be embossed with decorative designs.

The absorbent article may be packaged as unwrapped absorbent articles within a carton, box or bag. The consumer withdraws the ready-to-use article as needed. The absorbent article may also be individually packaged (each absorbent article encased within an overwrap).

Also contemplated by the present invention are asymmetrical and symmetrical absorbent articles having parallel longitudinal edges, dog bone- or peanut-shaped, as well as articles having a tapered construction for use with thong-style undergarments. From the foregoing description, one skilled in the art can ascertain the essential characteristics of this invention, and without departing from the spirit and scope thereof, can make various changes and modifications. Embodiments set forth by way of illustration are not intended as limitations on the variations possible in practicing the present invention.

### EXAMPLE

Specific inventive examples of the present invention are described below.

### Inventive Example #1

An example of a sanitary napkin according to the invention was constructed as follows. The body facing cover layer was constructed from a 16 gsm thermal bonded nonwoven material constructed from 100% hydrophilic polypropylene fibers, commercially available form Polystar Company, Salvador, Brazil under product code 142250. An absorbent core was arranged below the cover and was formed by the process described herein with reference to Figs. 8-12. The absorbent core has a plurality of first regions extending over a surface area of 1256.6 mm² (i.e. the summation of the area over which the first regions extend) and second region with a surface area of 10548.7 mm². Each of the plurality of first regions had a basis weight of 212 g/m² and the second region had a basis weight of 424 g/m². Each of the plurality of first regions 70 had a density of 0.035g/cm³ and the second region 72 had a density of 0.071 g/cm³. The absorbent core had a composition of about 93% by weight of pulp and about 7% by weight of superabsorbent polymer. The pulp was Golden Isles Fluff Pulp 420#HD 7% Moisture, commercially available from GP Cellulose, Brunswick, Georgia, USA. The superabsorbent polymer was Aqua Keep SA70N commercially available from Sumitomo Seika Chemicals Co., Ltd., Osaka, Japan. A barrier layer was arranged below the core and was formed from a 24 gsm polyethylene film commercially available from Clopay do Brasil, Jundiai, Brazil, under product code 113689. A layer of conventional positioning adhesive was applied to the garment facing surface of the barrier layer for attaching the napkin to an undergarment during use.

### Procedure for Measuring Relative Humidity

Absorbent articles according to the present invention provide enhanced internal breathability as compared to conventional prior art articles. The test method set forth below measures the humidity dissipation characteristics of an absorbent article which is indicative of the internal breathability characteristics of the article.

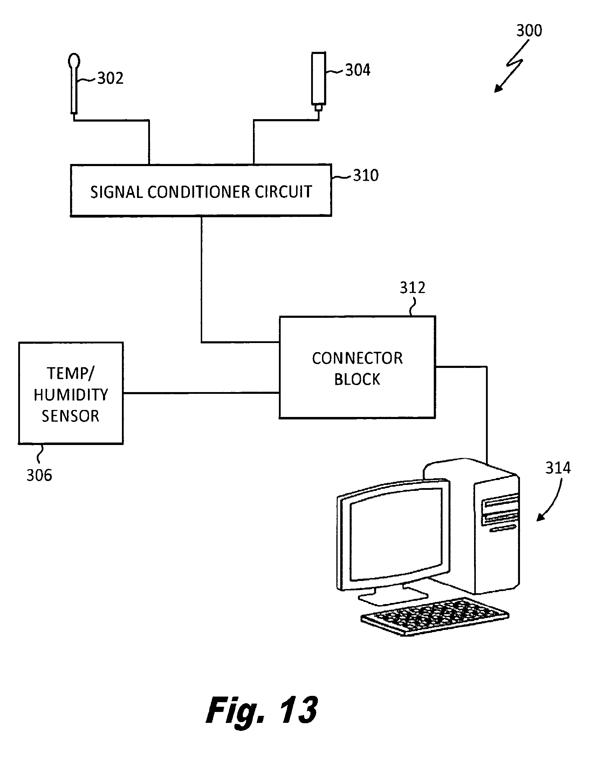

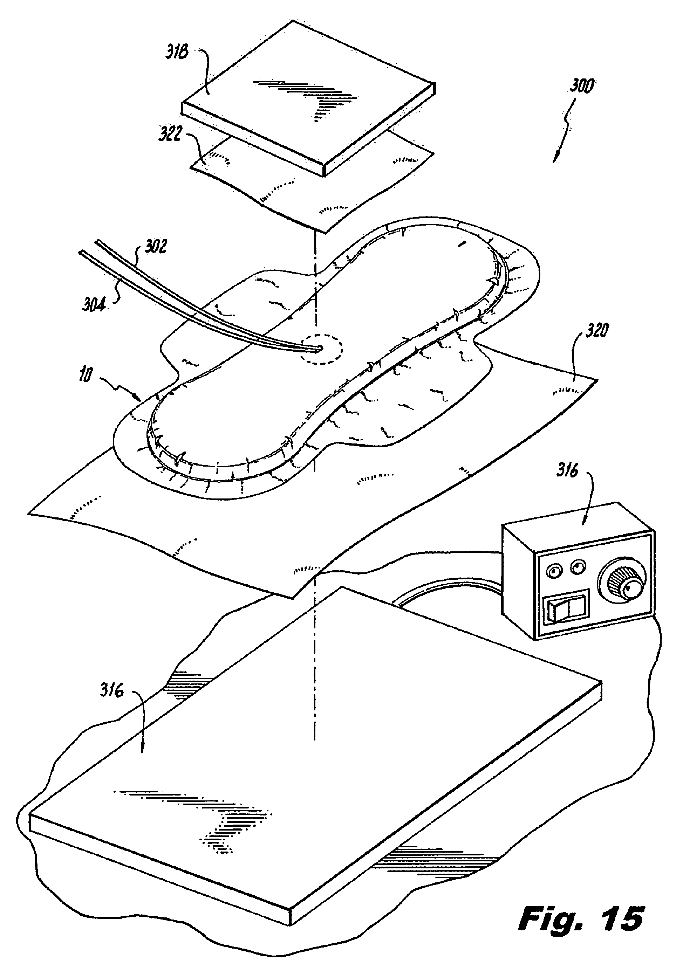

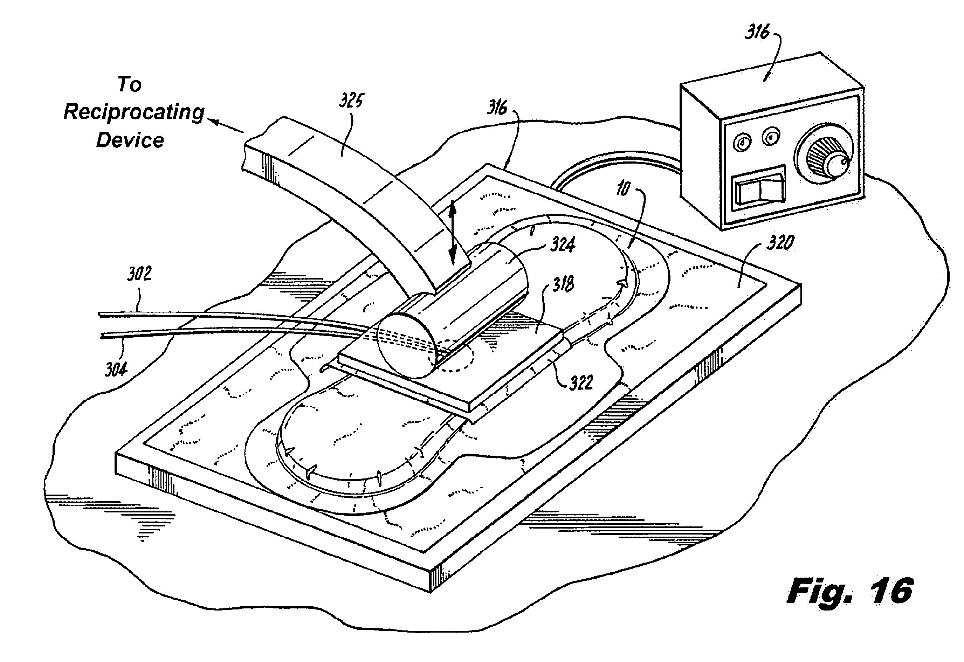

Reference is made to Figure 13 which schematically depicts an apparatus 300 for measuring the humidity dissipation characteristics of an absorbent article according to the test method set forth in detail below. The apparatus 300 generally includes a relative humidity microsensor 302 for measuring the relative humidity of an absorbent article, a microsensor 304 for measuring the temperature of an absorbent article, a temperature and humidity sensor 306 for measuring the temperature and relative humidity of a laboratory in which the test is being conducted, a signal conditioner 310, a connector block 312 and a computer 314 for recording the measured data. The apparatus 300 further includes a heating plate 316 as shown in Figs. 15 and 16.

Two acrylic plates 318 each having dimensions of 5.0 cm (length) by 5.0 cm (width) by 0.2 cm (thick) are used in the test method described below. One of the above described acrylic plates 318 is depicted in Fig. 15. A cotton panty 320, as shown in Fig. 15, is also required to perform the test method set forth below.

A suitable commercially available microsensor 302 is the relative humidity microsensor model HIH-400 manufactured by Honeywell International, Inc., Morristown, New Jersey.

A suitable commercially available microsensor 304 is temperature microsensor model NTC manufactured by BetaTherm, Inc., Hampton, Virginia.

The same commercially available temperature and relative humidity microsensors described above may be used as the sensor 306 to measure the temperature and relative humidity of the laboratory in which the test is being conducted.

The electronic interface 310 is a conventional signal conditioner circuit.

A suitable commercially available connector block 312 is connector block model NISCC-68 manufactured by National Instruments Corporation, Austin, Texas.

The computer 314 is a Microsoft Windows based system equipped with LabView, version 7.1, manufactured by National Instruments Corporation, Austin, Texas. The identified software is used to collect and process the transmitted data.

A suitable commercially available heating plate 316 is the Multi-Blok Heater, Model 2050, manufactured by Lab-Line Instruments, a subsidiary of Breanstead Thermolyne, Melrose Park, Illinois.

The cotton panty 320 used in the test method may be any conventional commercially available panty having a composition of at least 90% cotton.

As shown in Fig. 16, the apparatus 300 further includes a cylindrical mass 324 having an outer diameter of 3.0 cm, a length of 8.5 cm and a mass of 77.3 g. The cylindrical mass 324 may be constructed as an acrylic tube filled with sand or the like to achieve the required mass and sealed on each end thereof. The cylindrical mass 324 is connected to a rigid swing arm 325, which is in turn connected any suitable apparatus capable of moving the mass 324 in a repeating up and down vertical motion to thereby apply a repeating force to the acrylic plate 318 as shown in Fig. 16. The apparatus to which the mass 324 it attached, by means of the swing arm 325, should be selected such that the mass 324 applies a force of 12 g once per second to the acrylic plate 318. A suitable commercially available apparatus capable of moving the swing arm 325 and mass 324 in this manner, and applying the required force, is a thermostatic bath Haake SWB 20 Fisons TYP 000-8582/194015695002 KL DIN 12879 manufactured by Haake Fisons.

As shown in Fig. 15, one of the surfaces of the acrylic plate 318 is covered by a 5 cm (length) X 5 (width) cm X 0.1 cm (thickness) swatch of nonwoven material 322. The nowoven material 322 is attached to the acrylic plate 318 by applying 3.6 gsm (g/m²) adhesive (Pritt non-toxic Stick manufactured by Henkel Capital, S.A., Mexico) over a 25 cm² area to the acrylic plate facing surface of the nonwoven material 322. The nonwoven material 322 has a basis weight of 180 gsm and a composition of 100% wool fibers. A suitable commercially available material of this type is available from Indústria de Feltros Santa Fé Av. Antônio Bardella, 780, Cumbica, Guarulhos-SP Brazil.

Prior to conducting the test method set forth below the product specimens to be measured are conditioned by leaving them in a room that is 22°C, +/-2°C and 55%, +/-3.0% relative humidity for a period of twelve (12) hours. In addition, for each product specimen to be tested, two acrylic plates 318, with the nonwoven swatch of material 322 attached thereto, are conditioned by leaving them in a room that is 22°C, +/-2°C and 55%, +/- 3.0% relative humidity for a period of twelve (12) hours. Three identical product specimens are required for each product to be tested.

The test method described below should be conducted in a laboratory setting having a temperature in the range of between 22° C, +/- 2° C and a relative humidity of 55%, +/- 3.0%.

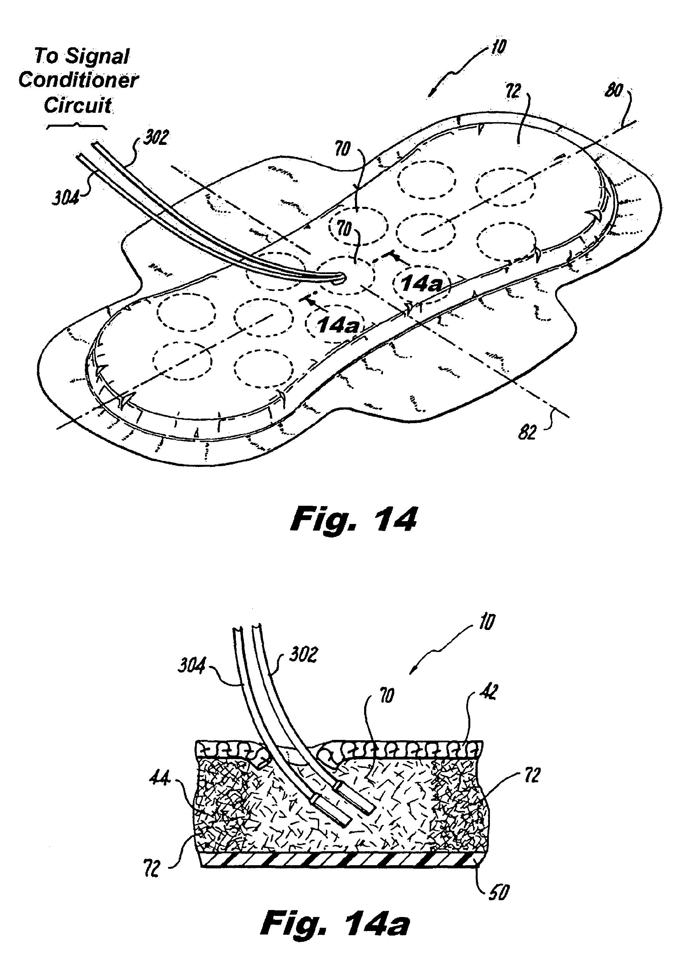

As shown in Figs. 14 and 14a, the test method is initiated by inserting the microsensor 302 and the microsensor 304 under the cover layer 42 and into the absorbent core layer 44 at the intersection of the longitudinally extending centerline 80 and transversely extending centerline 82 of the absorbent article 10. A small hole may be formed in the cover layer 42, if necessary, to facilitate the insertion of the microsensors 302 and 304.

The specific inventive example 10 tested had a construction as described above for "Inventive Example #1". The inventive example 10 included a first region 70 located at the intersection of the longitudinally extending centerline 80 and transversely extending centerline 82, thus the microsensors 302 and 304 were positioned within one of the first regions 70.

After the microsensors 302 and 304 are inserted under the cover layer 42 and into core layer 44 the napkin 10 is attached to the panty 320 by means of positioning adhesive located on the garment facing surface of the barrier layer 50. If the article to be tested does not include positioning adhesive the article may be attached to the panty 320 using conventional masking tape or the like.

After the napkin 10 is attached to the panty 320, the panty 320 is arranged on the heating plate 316, as shown in Figs. 15 and 16, such that the panty 320 is adjacent the top surface of the heating plate 316 and the napkin 10 faces away from the top surface of the heating plate 316. Thereafter one of the conditioned acrylic plates 318 is arranged on top of the napkin 10 such that the center of the plate 318 is arranged over the intersection of the longitudinally extending centerline 80 and transversely extending centerline 82 of the napkin 10. The plate 318 is arranged such that the nonwoven swatch of material 322 is placed in abutting face to face relationship with the top surface of the cover layer 42. The cylindrical mass 324 is then positioned such that the central axis thereof is aligned with the longitudinally extending centerline 80 of the napkin 10.

After the apparatus 300 is configured as described above, the movement of the cylindrical mass 324 is initiated and the relative humidity and temperature of the napkin 10 is monitored via the readout provided by the computer 314. The objective of this first step of the method is to obtain an equilibrium temperature and relative humidity within the napkin 10. Specifically, the objective is to obtain conditions within the napkin 10 such that the temperature of the napkin is between 36° and 38° C and the relative humidity of the napkin is between 25% to 30%. Equilibrium is established when the napkin 10 has a temperature between 36° and 38° C and a relative humidity between 25% to 30% for a period of one minute. The temperature of the napkin 10 may be increased, if necessary, to reach the required equilibrium temperature by means of the heating plate 316.

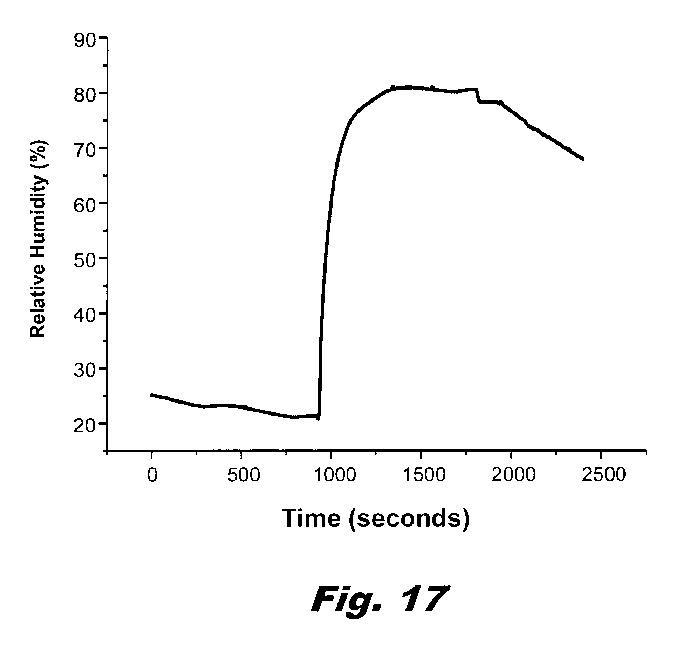

Once the equilibrium temperature and equilibrium relative humidity has been established in the napkin 10 as described above, the computer 314 and the LabView 7.1 software are used to begin collecting relative humidity data from the napkin 10. Data is collected for a fifteen minute period. After the initial fifteen minute period, the first plate 318 is removed and replaced with a new second plate 318, having the swatch of nonwoven material 322 attached thereto, that has been previously conditioned by leaving the plate 318 and material 322 in a room that is 22°C, +/-2°C and 55%, +/- 3.0% relative humidity for a period of twelve (12) hours. Prior to applying the second plate 28 to the napkin 40, 0.5 mL of water is applied to nonwoven material 36 using any conventional syringe. After the second plate 318 is applied relative humidity data for the napkin 10 is collected for an additional fifteen minute period. Thereafter, the second plate 318 is removed and relative humidity data for the napkin 10 is collected for an additional 10 minute period. Thus, relative humidity data is collected from the napkin 10 for a total of forty-five minutes. The relative humidity data collected from the napkin 10 is then used to generate a relative humidity (%) versus time (s) graph of the type shown in Fig. 17. The graph is generated using the data analysis and graphing software Origin 6.0 commercially available from OriginLab Corporation, Northampton, Massachusetts.

The graph shown in Fig. 17 shows the relative humidity data collected for Inventive Example #1 as described above. As will be described in greater detail below the graph of relative humidity shown in Fig. 17 is used to calculate the humidity dissipation value A_{RH} of the napkin. Absorbent articles according to the present invention preferably have A_{RH} greater than 4500, preferably greater than 5500 and most preferably greater than 6500.

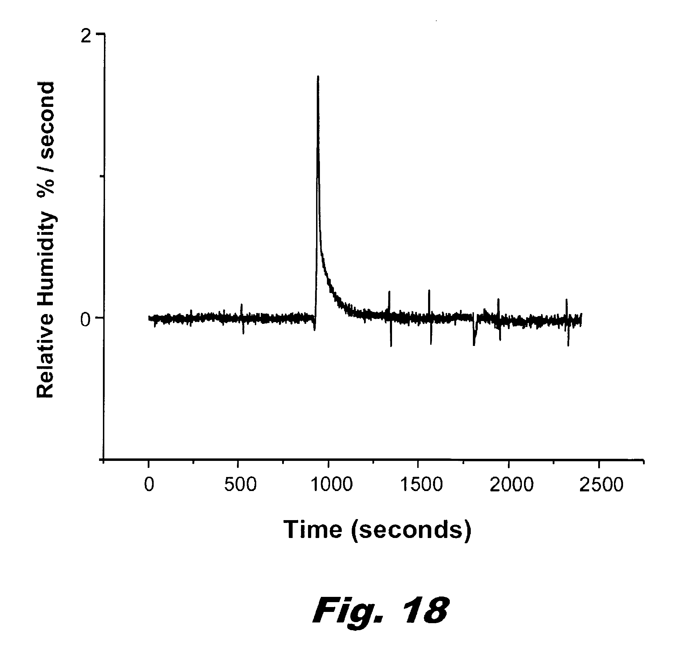

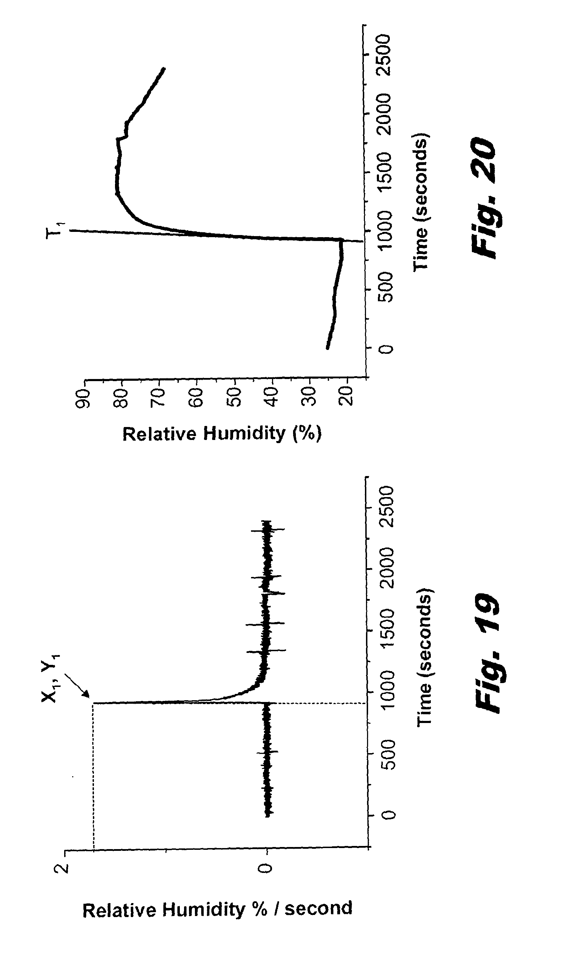

The A_{RH} calculation is performed as described below. First the differential of the graph shown in Fig. 17 is plotted to obtain a graph of the type shown in Fig. 18. Thereafter, as shown in Fig. 19, the maximum relative humidity %/second value, Y₁, is determined from the maximum value on the differential graph. Once the maximum value relative humidity %/second value, Y₁ is determined, the time at which this value occurs X₁ can be determined. Using the point defined by X₁ and Y₁, and the slope of the differential graph at this point, a first tangent line T₁ is obtained in time X₁ can be defined as shown in Fig. 20. The first tangent line T₁ is then transcribed on the graph shown in Fig. 17 as shown in Fig. 20. The tangent line T₁ is generated using the Origin 6.0 software.

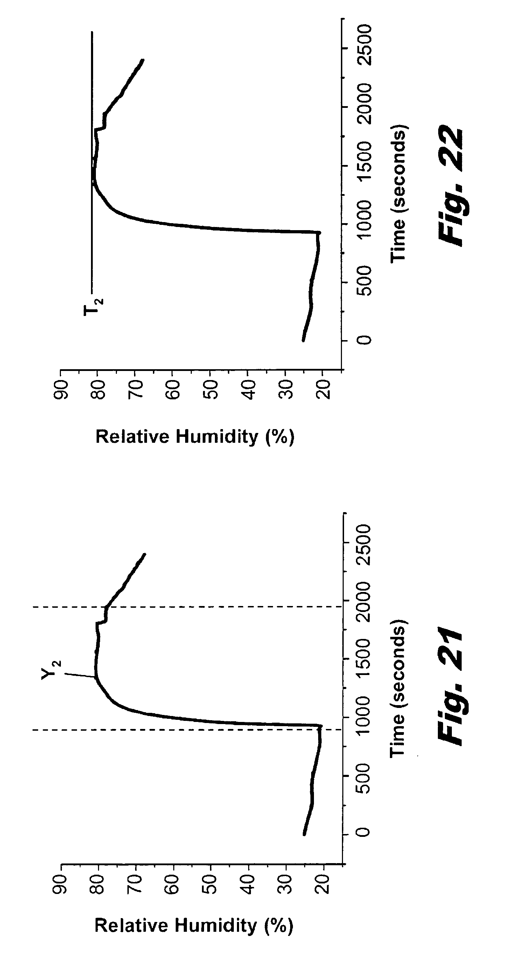

A second tangent line T₂ is determined by determining the maximum relative humidity % value, Y2, on the graph shown in Fig. 17 between 900 s and 1800 s, as shown in Fig. 21. Using this maximum relative humidity % value, Y₂, and a slope of zero, a second tangent line T₂ can be defined. The second tangent line T₂ is transcribed on the graph shown in Fig. 17 as shown in Fig. 22. The tangent line T₂ is generated using the Origin 6.0 software.

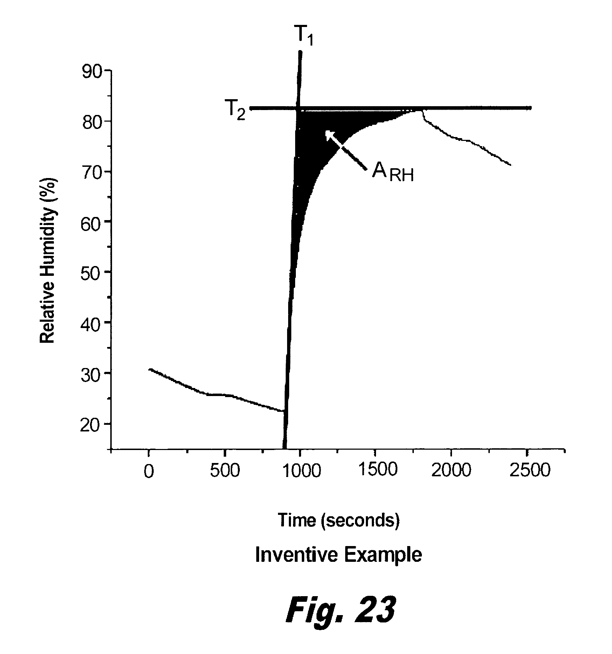

Once the first tangent line T₁ and second tangent line T₂ are transcribed on the graph shown in Fig. 17, as shown in Fig. 23, the area A_{RH} located between the graph line and first and second tangent lines is calculated using the Origin 6.0 software. The calculated area A_{RH} is inversely proportional to the relative humidity retention of the napkin 10. Stated another way the higher the A_{RH} the greater the humidity dissipation of the napkin 10. Thus, the higher the A_{RH} value the lower the relative humidity retention of the product and the cooler and more comfortable the product will feel during use.

The above described calculation is repeated for three identical product samples and an average A_{RH} is calculated. The average A_{RH} for Inventive Example #1 was calculated to be 6872.55 (%-second).

A commercially available product, Regular Sempre Livre, distributed by Johnson & Johnson do Brasil Ind. Com. Prod. para Saúde Ltda, Brazil, was tested according to the above described test method. The A_{RH} for the comparative product is shown in Figure 23. The average A_{RH} for the Regular Sempre Livre product was calculated to be 3985.72 (%-second).

In view of the above, absorbent articles according to the present invention provide superior relative humidity regulation properties as compared to prior art absorbent articles and thereby are capable of providing a more comfortable use experience.

Applications of the absorbent article according to the present invention for sanitary and other health-care uses can be accomplished by any sanitary protection, incontinence, medical and absorbent methods and techniques as are presently or prospectively known to those skilled in the art. Thus, it is intended that the present application cover the modifications and variations of this invention provided that they come within the scope of the appended claims and their equivalents.

## Claims

1. An absorbent article comprising:
a liquid pervious cover layer;
a liquid impervious barrier layer;
an absorbent core arranged between the cover layer and barrier layer, the absorbent core having a plurality of first regions and a second region, each one of said first regions being arranged in spaced relationship from each of the other first regions and each of the first regions being entirely surrounded by the second region;
the second region having a basis weight and each one of the plurality of first regions having a basis weight, wherein the basis weight of each of the first regions is less than the basis weight of the second region;
wherein the entire absorbent core, including the plurality of first regions and the second region, has a uniform thickness.

2. The absorbent article according to claim 1, wherein the plurality of first regions have a basis weight in the range of 7.5gsm to 555 gsm and wherein the second region has a basis weight in the range 150gsm and 650gsm.

3. The absorbent article according to claim 1 or claim 2, wherein the basis weight of each of the plurality of first regions is about 5% to 85% the basis weight of the second region.

4. The absorbent article according to any preceding claim, wherein the first regions extend over about 5% to 30% of the surface area of the core.

5. The absorbent article according to claim 4, wherein the second region extends over about 70% to about 95% of the surface area of the core.

6. The absorbent article according to any preceding claim, wherein the absorbent core comprises between about 75% to 100% cellulose fibers by weight and 0% to 25% superabsorbent by weight.

7. The absorbent article according to any preceding claim, wherein the plurality of first regions and the second region have the same identical material composition.

8. The absorbent article according to any preceding claim, wherein the absorbent core consists of a single layer of material.

9. The absorbent article according to any preceding claim, wherein each of the plurality of first regions has a density that is less than the density of the second region.

10. The absorbent article according to any preceding claim, wherein each of the first regions has a density in the range from about 0.017 g/cm³ to 0.200 g/cm³ and the second region has a density in the range of about 0.035 g/cm³ to 0.400 g/cm³.

11. The absorbent article according to any preceding claim, wherein each of the first regions has a density that is less than 80 % the density of the second region.

12. The absorbent article according to any preceding claim, wherein each of the first regions has a density that is less than 50 % the density of the second region.

13. The absorbent article according to any preceding claim, wherein the absorbent article has an average humidity dissipation value A_{RH} greater than 4500.

14. The absorbent article according to any preceding claim, wherein the absorbent article has an average humidity dissipation value A_{RH} greater than 5500.

15. The absorbent article according to any preceding claim, wherein the absorbent article has an average humidity dissipation value A_{RH} greater than 6500.
